# EUROPEAN PATENT APPLICATION

(11) **EP 4 834 235 A1**
(43) Date of publication of application: **04.11.2026**
(21) Application number: 24910993.5
(22) Date of filing: 22.12.2024
(51) Int. Cl.: A61B 6/58, A61B 6/03, G06T 5/80

(54) **NONLINEAR CORRECTION METHOD AND SYSTEM FOR DETECTOR, AND STATIC CT DEVICE**

(30) Priority: 30.12.2023 CN 202311866290
(71) Applicant: Nanovision (Nanjing) Technology Group Co., Ltd., Nanjing, Jiangsu 211102 (CN)
(72) Inventor: LIANG, Song, Beijing 100094 (CN); CHEN, Geng, Beijing 100094 (CN); CUI, Zhili, Beijing 100094 (CN); LI, Yunxiang, Beijing 100094 (CN)
(74) Representative: Wang, Bo
(86) International application number: PCT/CN2024/141258
(87) International publication number: WO 2025/140065

(57) **Abstract**

Disclosed are a nonlinear correction method and system for a detector, and a static CT device. The method comprises the following steps: arranging a phantom in a detection area of the static CT device, and making the rotating axis of the phantom parallel to the central axis of the static CT device; controlling a double-ring structure to rotate relative to the phantom, and collecting image data of the phantom in the relative rotation process; calculating a pixel difference value at each relative angle; drawing a fitting curve corresponding to each projection position; storing a curve coefficient of each fitting curve as a nonlinear correction coefficient of a projection image at a corresponding projection position; and on the basis of each nonlinear correction coefficient, carrying out nonlinear correction on the projection image at each projection position. In the method, polynomial fitting is carried out on a difference between a projection value at each relative angle and a projection value of a standard template to obtain a nonlinear correction table, and each projection position has enough statistical data, such that the nonlinear correction can achieve an ideal effect.

## Description

### BACKGROUND

### Technical Field

The present disclosure relates to a nonlinear correction method for a detector, also relates to a corresponding nonlinear correction system, further relates to a static CT device using the nonlinear correction method, and belongs to the technical field of radiation imaging.

### Related Art

Static CT devices face some challenges in terms of detector technologies, and these challenges affect image quality and diagnosis accuracy. First, due to small size and large distribution density of pixels of a detector, some pixels have poor response linearity. This leads to inconsistency of pixel response. Secondly, most existing photon-counting detectors form a large-area detector array through module splicing, and pixels at edges of the modules have worse linearity due to reasons such as a manufacturing process. In addition, most static CT devices use multi-source systems, and energy spectra of different ray sources are inconsistent, which further increases inconsistency between the ray sources. These inconsistency phenomena cause an artifact in a reconstructed CT image, and affect uniformity of CT values, to adversely affect image observation and result evaluation. Therefore, solving these inconsistency problems is crucial to development and application of the static CT devices.

### SUMMARY

A primary technical problem to be solved in the present disclosure is to provide a nonlinear correction method for a detector.

Another technical problem to be solved in the present disclosure is to provide a nonlinear correction system for a detector.

Still another technical problem to be solved in the present disclosure is to provide a static CT device using the foregoing nonlinear correction method.

To achieve the above-mentioned technical objectives, the disclosure adopts the following technical solutions:
According to a first aspect of embodiments of the present disclosure, a nonlinear correction method for a detector is provided, including the following steps:
arranging a phantom in a detection area of a static CT device, and making a rotating axis of the phantom parallel to a central axis of the static CT device, where a double-ring structure of the static CT device is rotatable relative to the phantom, and the double-ring structure is formed by a detector ring and a ray source ring that are relatively fixed;
controlling, according to a preset interval angle, the double-ring structure and the phantom to rotate relative to each other, to collect a projection image obtained after each ray source scans the phantom at any relative angle, where each projection image corresponds to a fixed projection position;
performing pre-correction on all the projection images, to respectively acquire pre-corrected images of all ray sources at relative angles;
performing, for any relative angle, pixel averaging processing on pre-corrected images formed by all ray sources at the relative angle in a relative rotation process, to acquire an average pixel value at the relative angle;
selecting any projection position and selecting any relative angle, acquiring a pixel value of a pre-corrected image corresponding to the projection position at the relative angle, and comparing the pixel value of the projection position with the average pixel value corresponding to the relative angle, to acquire a pixel difference value of the projection position at the relative angle; using the pixel difference value of the projection position at the relative angle and the pixel value of the projection position at the relative angle as a set of fitting data;
repeating the foregoing process to acquire fitting data of the projection position at each relative angle, to form a fitting data set corresponding to the projection position;
drawing a fitting curve corresponding to the projection position by using the fitting data set;
storing a curve coefficient of the fitting curve as a nonlinear correction coefficient of the projection image at the projection position; and
performing nonlinear correction on the projection image at the projection position based on the nonlinear correction coefficient.

Preferably, each projection position includes an equal quantity of pixel areas, a pixel value of the pre-corrected image corresponding to the projection position is a pixel value set, and the pixel value set includes a sub-pixel value corresponding to each pixel area;
an average pixel value at any relative angle is an average pixel set, and the average pixel set includes a sub-average pixel value corresponding to each pixel area at the relative angle, where
any relative angle is selected for each of the pixel areas, and a sub-pixel value of the pixel area at the relative angle is compared with a sub-average pixel value of the pixel area at the relative angle, to obtain a sub-pixel difference value of the pixel area at the relative angle; the sub-pixel difference value of the pixel area at the relative angle and the sub-pixel value of the pixel area at the relative angle are used as a set of sub-fitting data;
the foregoing process is repeated, to acquire sub-fitting data of the pixel area at each relative angle, and form a sub-fitting data set corresponding to the pixel area;
a sub-fitting curve corresponding to the pixel area is drawn by using the sub-fitting data set; and
the fitting curve of the projection position is formed by sub-fitting curves corresponding to all the pixel areas.

Preferably, the phantom includes a plurality of water phantoms with continuously varying sizes, and the water phantoms are separately arranged in a detection area of the static CT device; and
for any projection position, each water phantom corresponds to a fitting data set for drawing a fitting curve, and the fitting curve of the projection position is drawn by jointly using the plurality of fitting data sets.

Preferably, the phantom has a plurality of varying positions, and the plurality of varying positions at least include above a central axis of the static CT device, below the central axis of the static CT device, on a left side of the central axis of the static CT device, and on a right side of the central axis of the static CT device; and
for any projection position, the water phantom at each position corresponds to one fitting data set for drawing a fitting curve, and the fitting curve of the projection position is drawn by jointly using the plurality of fitting data sets.

Preferably, the controlling, according to a preset interval angle, the double-ring structure and the phantom to rotate relative to each other includes:
controlling the phantom to remain stationary, and controlling the double-ring structure to rotate at a constant speed according to the preset interval angle; or
controlling the double-ring structure to remain stationary, and controlling the phantom to rotate at a constant speed according to the preset interval angle.

Preferably, a process of drawing the fitting curve is as follows:
selecting any relative angle, and using a pixel value of the pre-corrected image of the projection position at the relative angle as an x-coordinate; using a pixel difference value of the projection position at the relative angle as a y-coordinate, and marking a scatter point in a planar coordinate system;
repeating the foregoing process until marking of all scatter points is completed; and
performing polynomial fitting on the marked scatter points by using x as an independent variable and y as a dependent variable, to draw the fitting curve.

Preferably, the pre-correction includes at least background correction, air correction, and scatter correction.

Preferably, a relative rotation angle between the double-ring structure and the phantom is at least 90°.

According to a second aspect of the embodiments of the present disclosure, a nonlinear correction system for a detector is provided, including a processor and a memory, where the processor reads a computer program in the memory, and is configured to perform the foregoing nonlinear correction method.

According to a third aspect of the embodiments of the present disclosure, a static CT device is provided, using the foregoing nonlinear correction method to perform image correction.

Compared with the related art, the present disclosure has the following technical effects:
1. The double-ring structure of the static CT device is rotatable relative to the arranged phantom, and image collection can be performed at an interval of an angle during scanning a phantom at each position, to ensure that a plurality of ray sources can collect images of the phantom at each relative angle, thereby enabling a standard template at each relative angle to be an ideal template.
2. Polynomial fitting is performed on a difference between a projection value at each relative angle and a projection value of a standard template to obtain a nonlinear correction table. In addition, because there is enough statistical data at each relative angle, the nonlinear correction can achieve an ideal effect.
3. Phantoms with different sizes at a plurality of positions are scanned, to enable each projection position to have enough data to perform polynomial fitting, to obtain a nonlinear characteristic of each projection position.

### BRIEF DESCRIPTION OF THE DRAWINGS

FIG. 1 is a flowchart of a nonlinear correction method according to a first embodiment of the present disclosure.
FIG. 2 is a schematic diagram in a state in which a double-ring structure and a phantom are not rotated relative to each other in a first embodiment of the present disclosure.
FIG. 3 is a schematic diagram in a state in which a double-ring structure and a phantom are rotated by 10 ° relative to each other in the first embodiment of the present disclosure.
FIG. 4 is a schematic diagram in a state in which a double-ring structure and a phantom are rotated by 180 ° relative to each other in the first embodiment of the present disclosure.
FIG. 5 is a flowchart of a nonlinear correction method according to a second embodiment of the present disclosure.
FIG. 6 is a flowchart of a nonlinear correction method according to a third embodiment of the present disclosure.
FIG. 7 is a flowchart of a nonlinear correction method according to a fourth embodiment of the present disclosure.
FIG. 8 is a structural diagram of a nonlinear correction system according to a fifth embodiment of the present disclosure.

### DETAILED DESCRIPTION

The following describes technical content of the present disclosure in detail with reference to accompanying drawings and specific embodiments.

A core technical idea of this embodiment of the present disclosure lies in that: a double-ring structure of a static CT device is rotatable relative to an arranged phantom, and image collection can be performed at an interval of an angle during scanning a phantom at each position, to ensure that a plurality of ray sources can collect images of the phantom at each relative angle, thereby enabling a standard template at each relative angle to be an ideal template. Finally, polynomial fitting is performed on a difference between a projection value at each relative angle and a projection value of a standard template to obtain a nonlinear correction table. Because there is enough statistical data at each relative angle, the nonlinear correction can achieve an ideal effect.

### First embodiment

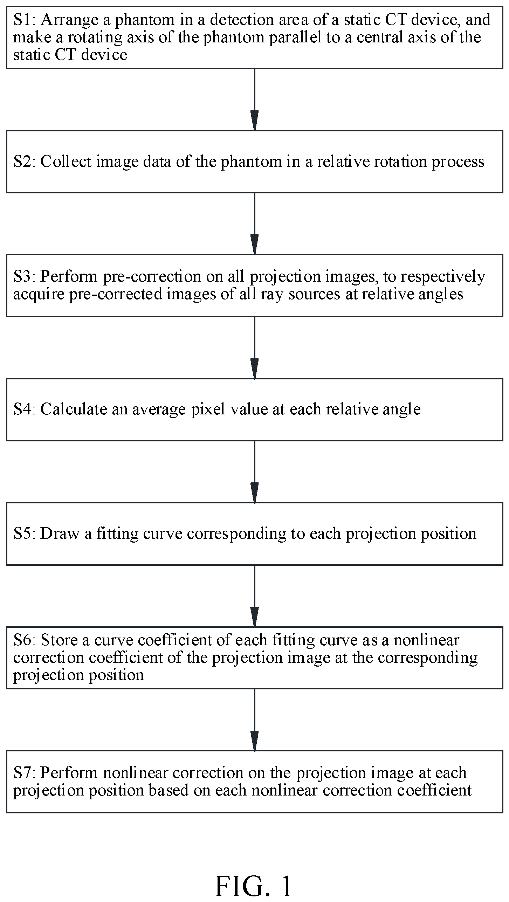

As shown in FIG. 1, a nonlinear correction method for a detector according to a first embodiment of the present disclosure specifically includes steps S1 to S7:
S1: Arrange a phantom in a detection area of a static CT device, and make the rotating axis of the phantom parallel to the central axis of the static CT device.

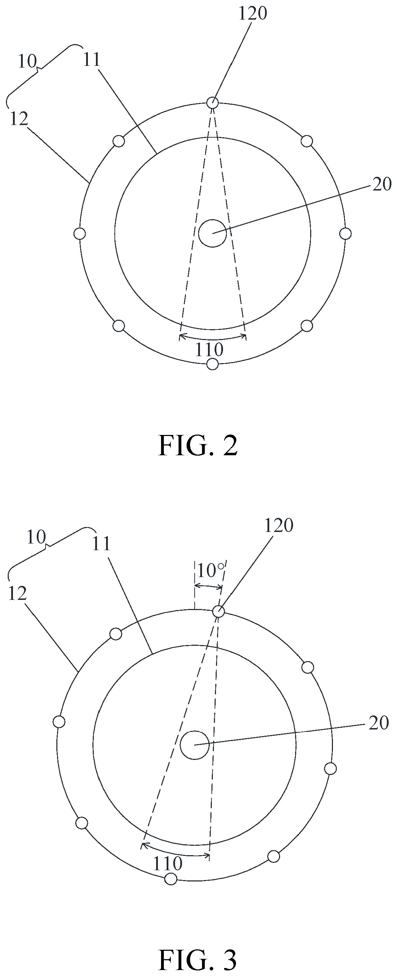

Specifically, as shown in FIG. 2, which only shows a double-ring structure 10 of a static CT device. The double-ring structure 10 includes a detector ring 11 and a ray source ring 12 that are relatively fixed. The rotating axis of the detector ring 11 is the central axis of the static CT device. The phantom 20 is preferably a cylindrical water phantom but is not limited to a shape and a type of the phantom 20. The phantom 20 is arranged in an annular area of the detector 11. In addition, the rotating axis of the phantom 20 is parallel to the central axis of the static CT device. Therefore, the phantom 20 can be scanned by using rays emitted from the ray source ring 12, and an image is formed on the detector ring 11. It may be understood that, in this embodiment, an arrangement position of the phantom 20 is not strictly required, provided that the position is approximately parallel to the central axis of the static CT device. An optimal position is that the rotating axis of the phantom 20 coincides with the central axis of the static CT device, and another position may alternatively be used.

In addition, in this embodiment, the double-ring structure 10 and the phantom 20 may rotate relative to each other, to perform image collection at an interval of an angle during scanning of the phantom 20 at each position. It may be understood that, relative rotation of the double-ring structure 10 and the phantom 20 may be implemented in the following manner:
In a first manner, the phantom 20 remains stationary, and the double-ring structure 10 rotates at a constant speed according to a preset interval angle (for example, 1°).

In a second manner, the double-ring structure 10 remains stationary, and the phantom 20 rotates at a constant speed according to a preset interval angle (for example, 1°).

Certainly, in another embodiment, both the double-ring structure 10 and the phantom 20 may alternatively rotate, to jointly rotate by the preset interval angle (for example, 1°).

In addition, to ensure enough data during subsequent polynomial fitting (which is described in detail below), in this embodiment, a relative rotation angle between the double-ring structure 10 and the phantom 20 is at least 90°, preferably 90° to 180°, and may exceed 180°. In principle, a larger angle indicates a better statistic. However, a data volume needing to be processed is larger.

S2: Collect image data of the phantom 20 in a relative rotation process.

Specifically, the double-ring structure 10 and the phantom 20 are controlled to rotate relative to each other according to a preset interval angle, to collect a projection image obtained after each ray source scans the phantom 20 at any relative angle. Positions of the detector ring 11 and the ray source ring 12 are relatively fixed. Therefore, each ray source corresponds to a fixed projection position. In addition, in this embodiment, each projection position includes an equal quantity of pixel areas (that is, the projection image includes a plurality of pixel blocks, and quantities of the pixel blocks are equal).

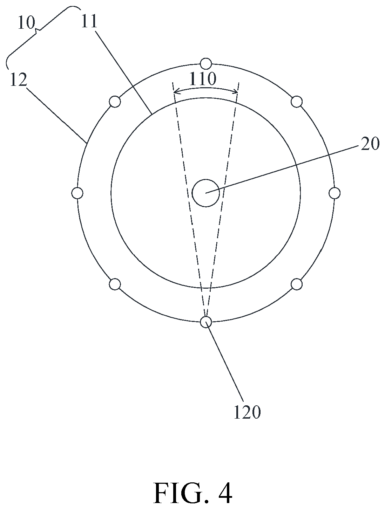

For ease of understanding, in this embodiment, as shown in FIG. 2 to FIG. 4, a description is provided by using an example in which a relative rotation angle between the double-ring structure 10 and the phantom 20 is 180°, a preset interval angle is 1°, and the ray source ring 12 has eight ray sources 120 corresponding to eight projection positions 110 on the detector ring 11, and each projection position 110 corresponds to 10 pixel areas.

It may be understood that, each time the double-ring structure 10 and the phantom 20 rotate relative to each other by 1°, after all ray sources (that is, the eight ray sources) on the ray source ring 12 scan the phantom 20, a group of projection images (eight projection images) are formed on the detector ring 11. Therefore, after the double-ring structure 10 and the phantom 20 rotate relative to each other by 180 °, in a relative rotation process of the double-ring structure 10 and the phantom 20, 180 groups of projection images need to be collected in total. Correspondingly, for each ray source, 180 projection images need to be collected in the relative rotation process between the double-ring structure 10 and the phantom 20.

S3: Perform pre-correction on all projection images, to respectively acquire pre-corrected images of all ray sources at relative angles.

Specifically, the pre-correction includes all correction processes except the nonlinear correction, for example, background correction, air correction, and scatter correction. A specific correction manner may be adaptively selected according to a requirement, and is not specifically limited herein.

It may be understood that, in this embodiment, if 180 groups of projection images are collected based on step S2, pre-correction needs to be performed on all the 180 groups of projection images.

S4: Calculate an average pixel value at each relative angle.

Specifically, steps S41 to S43 are included.

S41: Select any relative angle.

For example, if the relative angle is selected as 1°, it indicates that the double-ring structure 10 and the phantom 20 rotate relative to each other by 1°. If the relative angle is selected as 2°, it indicates that the double-ring structure 10 and the phantom 20 rotate relative to each other by 2°.

S42: Perform, for any relative angle, pixel averaging processing on pre-corrected images formed by all ray sources at the relative angle in a relative rotation process, to acquire an average pixel value at the relative angle.

Specifically, a description is provided by using FIG. 2 as a reference. It is assumed that a ray source marked as 120 in FIG. 2 is defined as a ray source No. 1, and other seven ray sources are numbered as 2 to 8 anticlockwise. After the relative angle is selected as 1°, in a process in which the double-ring structure 10 and the phantom 20 rotate relative to each other by 180 °, four ray sources numbered 1 to 4 in total contribute to imaging at 1°.

Therefore, an average pixel value at 1° may be obtained by averaging the pre-corrected images formed by the four ray sources numbered 1 to 4 at 1°. In addition, it is to be understood that because one pre-corrected image has 10 pixels, the average pixel value herein refers to an average pixel set, and the average pixel set includes 10 sub-average pixel values corresponding to 10 pixel areas.

S43: Repeat steps S41 to S42 until an average pixel value at all relative angles is obtained.

S5: Draw a fitting curve corresponding to each projection position.

Specifically, steps S51 to S56 are included.

S51: Select any projection position.

It may be understood that, in this embodiment, based on eight ray sources corresponding to eight projection positions, one of the eight projection positions needs to be selected, to draw a fitting curve for the projection position.

In addition, it may be understood that, because the projection position in this embodiment corresponds to 10 pixel areas, actually, drawing the fitting curve for the projection position is separately drawing sub-fitting curves for the 10 pixel areas, and the 10 sub-fitting curves jointly form the fitting curve of the projection position.

S52: Select any relative angle and acquire a pixel value of a pre-corrected image corresponding to the projection position at the relative angle.

Specifically, after the projection position is determined based on step S51, a corresponding ray source may be determined by using the projection position, and then projection images of the ray source at all relative angles (that is, 180 relative angles) may be obtained based on the data in step S2, to further obtain 180 pixel values corresponding to the projection position.

It may be understood that because the projection image has 10 pixel blocks, a pixel value corresponding to the projection position herein is a pixel value set, and the pixel value set includes 10 sub-pixel values respectively corresponding to the 10 pixel blocks.

S53: Acquire a pixel difference value of the projection position at the relative angle.

Specifically, after the pixel values of the projection position at the relative angle (that is, the sub-pixel values of the 10 pixel blocks) are acquired based on step S52, the sub-pixel values of the 10 pixel blocks are respectively compared with respective corresponding sub-average pixel values (refer to step S42), to respectively obtain 10 sub-pixel difference values of the 10 pixel blocks at the relative angle. The 10 sub-pixel difference values jointly form a pixel difference value of the projection position at the relative angle.

Therefore, for a pixel area of the projection position, after a relative angle is selected, a sub-pixel value of the pixel area at the relative angle and a sub-pixel difference value of the pixel area at the relative angle are used as a set of sub-fitting data. One projection position corresponds to a total of 10 sets of sub-fitting data at one relative angle.

S54: Repeat the foregoing steps S52 to S53, to acquire fitting data of the projection position at each relative angle, to form a fitting data set corresponding to the projection position.

It may be understood that, in an embodiment of the present disclosure, a relative rotation angle between the double-ring structure and the phantom is 180°, and each rotation interval is 1°. Therefore, for one projection position, fitting data at 180 relative angles in total needs to be collected, to form a fitting data set corresponding to the projection position. In addition, it may be understood that the fitting data set corresponding to the projection position includes 10 sub-fitting data sets corresponding to 10 pixel areas.

S55: Draw a fitting curve corresponding to the projection position.

Specifically, steps S551 to S552 are included.

S531: Standard scatter point.

Specifically, any relative angle (for example: 1°) is selected, and a pixel value of the pre-corrected image of the projection position at 1° is used as an x-coordinate. The x-coordinate herein includes 10 coordinate values in total, which respectively correspond to x-coordinates of 10 pixel areas.

In addition, a pixel difference value of the projection position at 1° is used as a y-coordinate. The y-coordinate herein includes 10 coordinate values in total, which respectively correspond to y-coordinates of 10 pixel areas.

A scatter point is marked in a planar coordinate system for each pixel area of the projection position.

The foregoing process is repeated until marking of all scatter points is completed.

It may be understood that after the relative angle changes, both the x-coordinate and the y-coordinate change correspondingly. Therefore, for any pixel area of the projection position, 180 scatter points are marked in the planar coordinate system.

S552: Polynomial fitting.

Specifically, for each pixel area of the projection position, a sub-pixel value of the pixel area is used as an independent variable x, a sub-pixel difference value of the pixel area is used as a dependent variable y, and polynomial fitting is performed on the marked 180 scatter points, to form a sub-fitting curve corresponding to the pixel area.

After polynomial fitting of the 10 pixel areas is completed sequentially, 10 sub-fitting curves are formed, to jointly form a fitting curve of the projection position.

S56: Repeat the foregoing steps S51 to S55 to obtain a fitting curve of each projection position.

It may be understood that, in this embodiment, because there are eight projection positions, and each projection position corresponds to 10 pixel areas, 8*10=80 sub-fitting curves need to be formed.

S6: Store a curve coefficient of each fitting curve as a nonlinear correction coefficient of the projection image at the corresponding projection position.

It may be understood that, in this embodiment, curve coefficients of 80 sub-fitting curves need to be stored in total, to form 80 nonlinear correction coefficients corresponding to 80 pixel areas.

S7: Perform nonlinear correction on the projection image at each projection position based on each nonlinear correction coefficient.

Specifically, calculation is performed according to the nonlinear correction coefficients corresponding to the pixel areas at each projection position and the pixel values corresponding to the pixel areas, to obtain a pixel difference value, and then pixel subtraction is performed, thereby completing nonlinear correction of a pixel area.

After the nonlinear correction of all pixel areas is completed, the nonlinear correction may be performed on the projection image at each projection position.

### Second embodiment

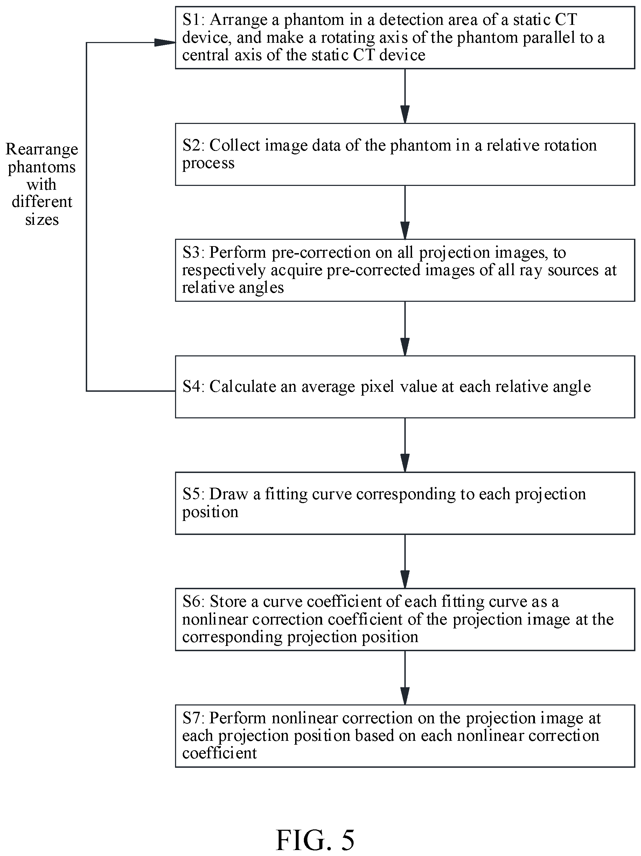

As shown in FIG. 5, based on the foregoing first embodiment, a second embodiment of the present disclosure further provides a nonlinear correction method for a detector. A difference between the first embodiment and the second embodiment lies in different quantities of phantoms 20.

Specifically, in this embodiment, the phantom 20 includes a plurality of water phantoms with continuously varying sizes, and the water phantoms are separately arranged in a detection area of the static CT device. It may be understood that, in this embodiment, for any projection position, one fitting data set can be acquired by using each water phantom. Therefore, before polynomial fitting is performed, each water phantom needs to be arranged in the detection area of the static CT device, to acquire n fitting data sets, where n is a quantity of the water phantoms.

Therefore, a data volume during polynomial fitting can be increased by using a plurality of water phantoms with continuously varying sizes, to improve fitting precision and improve an effect of nonlinear correction.

Other than the above difference, other steps in this embodiment are the same as those in the first embodiment, and details are not described again.

### Third embodiment

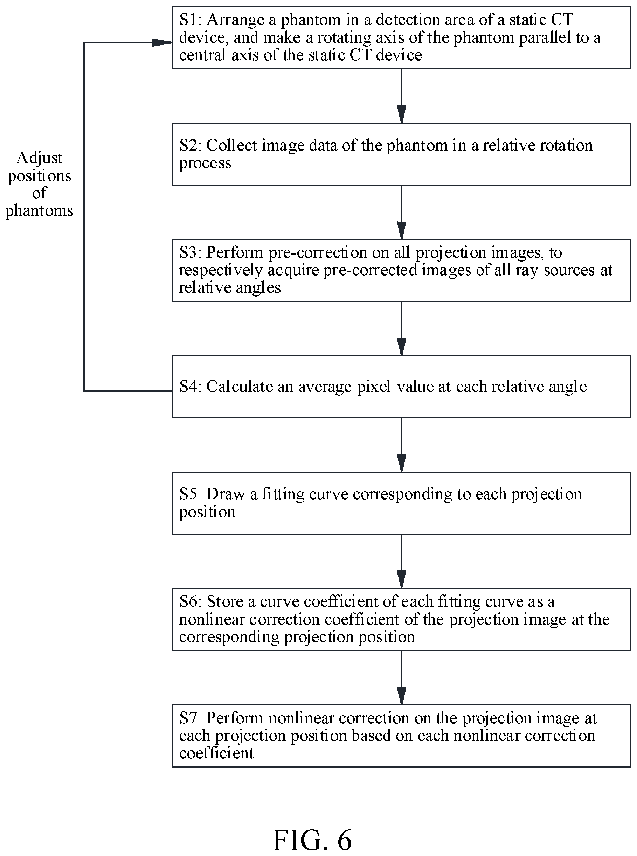

As shown in FIG. 6, based on the foregoing first embodiment, a second embodiment of the present disclosure further provides a nonlinear correction method for a detector. A difference between the first embodiment and the second embodiment lies in that positions of the phantoms 20 are variable.

Specifically, in this embodiment, the phantom has a plurality of varying positions. The plurality of varying positions include at least above a central axis of the static CT device, below the central axis of the static CT device, on a left side of the central axis of the static CT device, and on a right side of the central axis of the static CT device, and certainly may be positions such as top left, bottom left, top right, and bottom right of the central axis of the static CT device. Specifically, the plurality of varying positions may be adaptively set according to requirements.

It may be understood that for any projection position, each varying position corresponds to one fitting data set. Therefore, before polynomial fitting is performed, the water phantom needs to be adjusted to different positions, to obtain m fitting data sets, where m is a quantity of variable positions of the phantom.

Therefore, a data volume during polynomial fitting can be increased by using the phantom with varying positions, to improve fitting precision and improve an effect of nonlinear correction.

Other than the above difference, other steps in this embodiment are the same as those in the first embodiment, and details are not described again.

### Fourth embodiment

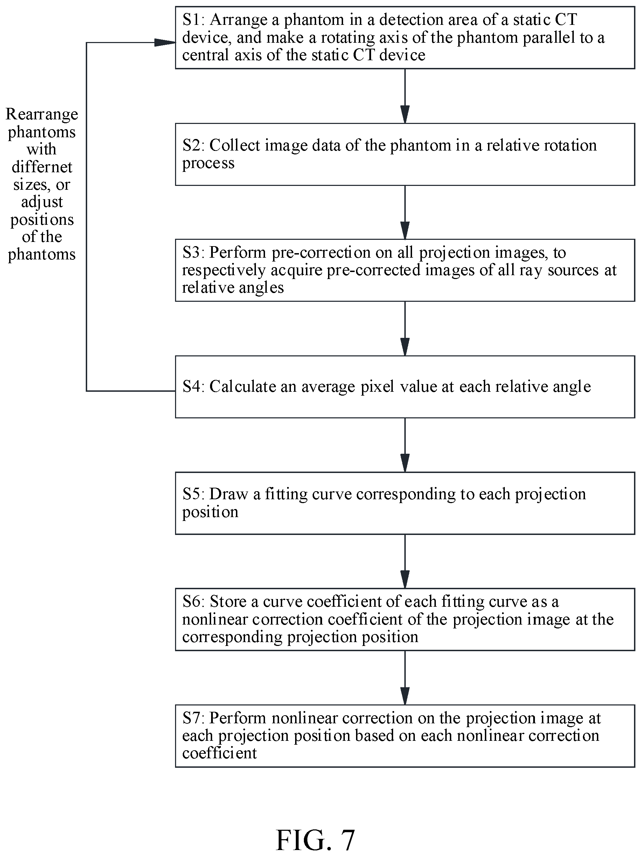

As shown in FIG. 7, based on the foregoing first embodiment, a second embodiment of the present disclosure further provides a nonlinear correction method for a detector. A difference between the first embodiment and the second embodiment lies in that the phantoms 20 have different quantities and variable positions.

In this embodiment, the phantom 20 includes n water phantoms with continuously varying sizes, and each water phantom has m varying positions. Therefore, before polynomial fitting, m*n fitting data sets can be acquired, thereby further improving fitting precision and improving an effect of nonlinear correction.

Other than the above difference, other steps in this embodiment are the same as those in the first embodiment, and details are not described again.

### Fifth embodiment

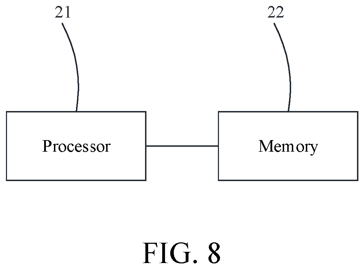

As shown in FIG. 8, based on the foregoing nonlinear correction method, the present disclosure further provides a nonlinear correction system for a detector. The nonlinear correction system includes one or more processors 21 and a memory 22. The memory 22 is coupled to the processor 21 and is configured to store one or more programs. When the program is executed by the processor 21, the processor 21 is enabled to implement the nonlinear correction system in the foregoing embodiments.

The processor 21 is configured to control an overall operation of the nonlinear correction system, to complete all or some of the steps of the foregoing nonlinear correction method. The processor 21 may be a central processing unit (CPU), a graphics processing unit (GPU), a field programmable gate array (FPGA), an application-specific integrated circuit (ASIC), a digital signal processing (DSP) chip, or the like. The memory 22 is configured to store various types of data to support operations of the nonlinear correction system. The data may include, for example, instructions of any application or method for operations in the nonlinear correction system, and data related to the application. The memory 22 may be implemented by any type of volatile or non-volatile storage devices or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, and a flash memory.

In an exemplary embodiment, the foregoing nonlinear correction system may be specifically implemented by a computer chip or an entity, or implemented by a product having a certain function, and is configured to perform the foregoing nonlinear correction method, and achieve the same technical effect as the foregoing method. In a typical embodiment, the system is a computer. Specifically, the computer may be, for example, a personal computer, a laptop computer, an on-board human-computer interaction device, a cellular phone, a camera phone, a smartphone, a personal digital assistant, a media player, a navigation device, an email device, a game console, a tablet computer, a wearable device, or any combination of these devices.

In another exemplary embodiment, the present disclosure further provides a computer-readable storage medium including program instructions. The program instructions, when executed by a processor, implement the steps of the nonlinear correction method in any one of the foregoing embodiments. For example, the computer-readable storage medium may be the foregoing memory including the program instructions, and the foregoing program instructions may be executed by the processor of the nonlinear correction system to complete the foregoing nonlinear correction method for a detector, and achieve the same technical effect as the foregoing method.

### Sixth embodiment

Based on the foregoing first embodiment to fourth embodiment, a sixth embodiment of the present disclosure further provides a static CT device. The static CT device performs image correction by using the nonlinear correction method in any one of the first embodiment to the fourth embodiment.

It is to be noted that the above plurality of embodiments are merely examples for description. Technical solutions of the embodiments may be combined, and all combinations fall within the protection scope of the present disclosure.

It is to be understood that orientations or positional relationships indicated by terms "thickness", "upper", "lower", "horizontal", and the like are the orientations or positional relationships shown based on the drawings, and are merely for the convenience of describing the present disclosure and simplifying the description, rather than indicating or implying that the devices or elements must have particular orientations, and constructed and operated in particular orientations. Therefore, this cannot be construed as a limitation to the present disclosure.

In addition, the terms "first" and "second" are used for descriptive purposes only and cannot be understood as indicating or implying relative importance or implicitly indicating the number of indicated technical features. Therefore, features defined by "first" and "second" may explicitly or implicitly include one or more of the features. In the descriptions of the present disclosure, unless otherwise explicitly specified, "a plurality of" means two or more than two.

The foregoing describes the nonlinear correction method and system for a detector, and the static CT device according to the present disclosure in detail. A person of ordinary skill in the art, any obvious change made to the present disclosure without departing from the essence of the present disclosure will constitute a violation of the patent right of the present disclosure and take corresponding legal responsibility.

## Claims

1. A nonlinear correction method for a detector, comprising the following steps:
arranging a phantom in a detection area of a static CT device, and making a rotating axis of the phantom parallel to a central axis of the static CT device, wherein a double-ring structure of the static CT device is rotatable relative to the phantom, and the double-ring structure is formed by a detector ring and a ray source ring that are relatively fixed;
controlling, according to a preset interval angle, the double-ring structure and the phantom to rotate relative to each other, to collect a projection image obtained after each ray source scans the phantom at any relative angle, wherein each projection image corresponds to a fixed projection position;
performing pre-correction on all the projection images, to respectively acquire pre-corrected images of all ray sources at relative angles;
performing, for any relative angle, pixel averaging processing on pre-corrected images formed by all ray sources at the relative angle in a relative rotation process, to acquire an average pixel value at the relative angle;
selecting any projection position and selecting any relative angle, acquiring a pixel value of a pre-corrected image corresponding to the projection position at the relative angle, and comparing the pixel value of the projection position with the average pixel value corresponding to the relative angle, to acquire a pixel difference value of the projection position at the relative angle; in addition, using the pixel difference value of the projection position at the relative angle and the pixel value of the projection position at the relative angle as a set of fitting data;
repeating the foregoing process to acquire fitting data of the projection position at each relative angle, to form a fitting data set corresponding to the projection position;
drawing a fitting curve corresponding to the projection position by using the fitting data set;
storing a curve coefficient of the fitting curve as a nonlinear correction coefficient of the projection image at the projection position; and
performing nonlinear correction on the projection image at the projection position based on the nonlinear correction coefficient.

2. The nonlinear correction method according to claim 1, wherein
each projection position comprises an equal quantity of pixel areas, a pixel value of the pre-corrected image corresponding to the projection position is a pixel value set, and the pixel value set comprises a sub-pixel value corresponding to each pixel area;
an average pixel value at any relative angle is an average pixel set, and the average pixel set comprises a sub-average pixel value corresponding to each pixel area at the relative angle, wherein
any relative angle is selected for each of the pixel areas, and a sub-pixel value of the pixel area at the relative angle is compared with a sub-average pixel value of the pixel area at the relative angle, to obtain a sub-pixel difference value of the pixel area at the relative angle; the sub-pixel difference value of the pixel area at the relative angle and the sub-pixel value of the pixel area at the relative angle are used as a set of sub-fitting data;
the foregoing process is repeated, to acquire sub-fitting data of the pixel area at each relative angle, and form a sub-fitting data set corresponding to the pixel area;
a sub-fitting curve corresponding to the pixel area is drawn by using the sub-fitting data set; and
the fitting curve of the projection position is formed by sub-fitting curves corresponding to all the pixel areas.

3. The nonlinear correction method according to claim 1, wherein
the phantom comprises a plurality of water phantoms with continuously varying sizes, and the water phantoms are separately arranged in a detection area of the static CT device; and
for any projection position, each water phantom corresponds to a fitting data set for drawing a fitting curve, and the fitting curve of the projection position is drawn by jointly using the plurality of fitting data sets.

4. The nonlinear correction method according to claim 1, wherein
the phantom has a plurality of varying positions, and the plurality of varying positions at least comprise above a central axis of the static CT device, below the central axis of the static CT device, on a left side of the central axis of the static CT device, and on a right side of the central axis of the static CT device; and
for any projection position, the water phantom at each position corresponds to one fitting data set for drawing a fitting curve, and the fitting curve of the projection position is drawn by jointly using the plurality of fitting data sets.

5. The nonlinear correction method according to claim 1, wherein the controlling, according to a preset interval angle, the double-ring structure and the phantom to rotate relative to each other comprises:
controlling the phantom to remain stationary, and controlling the double-ring structure to rotate at a constant speed according to the preset interval angle; or
controlling the double-ring structure to remain stationary, and controlling the phantom to rotate at a constant speed according to the preset interval angle.

6. The nonlinear correction method according to claim 1, wherein a drawing process of the fitting curve is as follows:
selecting any relative angle, and using a pixel value of the pre-corrected image of the projection position at the relative angle as an x-coordinate; using a pixel difference value of the projection position at the relative angle as a y-coordinate, and marking a scatter point in a planar coordinate system;
repeating the foregoing process until marking of all scatter points is completed; and
performing polynomial fitting on the marked scatter points by using x as an independent variable and y as a dependent variable, to draw the fitting curve.

7. The nonlinear correction method according to claim 1, wherein
the pre-correction comprises at least background correction, air correction, and scatter correction.

8. The nonlinear correction method according to claim 1, wherein
a relative rotation angle between the double-ring structure and the phantom is at least 90°.

9. A nonlinear correction system for a detector, comprising a processor and a memory, wherein the processor reads a computer program in the memory, and is configured to perform the nonlinear correction method according to any one of claims 1 to 8.

10. A static CT device, configured to perform image correction by using the nonlinear correction method according to any one of claims 1 to 8.
